# EUROPEAN PATENT SPECIFICATION

(11) **EP 1 586 315 B1**
(45) Date of publication and mention of the grant of the patent: **14.04.2010**
(21) Application number: 03782959.5
(22) Date of filing: 26.12.2003
(51) Int. Cl.: A61K 31/196, A61K 31/198, A61K 31/375, A61K 9/08, A61K 9/20, A61K 8/00, A61Q 1/00, A61P 17/00

(54) **SKIN LIGHTENING COMPOSITION**
ZUSAMMENSETZUNG ZUR HAUTAUFHELLUNG
COMPOSITION ECLAIRCISSANT LA PEAU

(30) Priority: 27.12.2002 JP 2002381304
(43) Date of publication of application: 19.10.2005
(73) Proprietor: Daiichi Sankyo Healthcare Co., Ltd., Chuo-ku Tokyo 103-8541 (JP)
(72) Inventor: MORIMOTO, Yoshinobu c/o Daiichi Pharmaceutical, Chuo-ku, Tokyo 103-8234 (JP); WATAKE, Asami c/o Daiichi Pharmaceutical, Chuo-ku, Tokyo 103-8234 (JP)
(74) Representative: Reitstötter - Kinzebach
(86) International application number: PCT/JP2003/017050
(87) International publication number: WO 2004/060364

(56) References cited:
- EP-A1- 1 002 515
- CA-A1- 2 086 565
- JP-A- 6 080 564
- JP-A- 11 092 326
- JP-A- 54 138 130
- JP-A- 59 128 320

## Description

### TECHNICAL FIELD

The present invention relates to a novel composition, more particularly a composition for whitening (whitening agent).

### BACKGROUND OF THE INVENTION

Pigmentations such as chloasmas, freckles, sunburn, dark skin and melanoderma caused by a drug such as steroid are generated by excess deposition of melanin pigment in the skin. It is known that biosynthesis of melanin occurs in a cytoplasmic granule, melanosome, in the melanocyte via a complex pathway in which tyrosine is oxidized by tyrosinase to cause biosynthesis of dopa and dopaquinone, and the dopaquinone is converted into indolequinone or the like due to auto-oxidation by ultraviolet rays. Particularly, such pigmentations are not preferable for women from the beauty point of view.

Known preventive and therapeutic agents for pigmentations (whitening agents) include L-ascorbic acid and its derivatives (cf. JP-A-49-86554), kojic acid (cf. JP-A-53-3538), L-cysteine (cf. JP-A-59-128320), arbutin (cf. JP-A-60-56912), a bearberry (*Arctostaphylos uva-ursi*) and its extracts (cf. JP-A-6-166609), an admixture of tranexamic acid and ascorbic acid (cf. JP-A-4-243 825 corresponding to CA 2 086 565 A1) and the like. JP-A-6 080 564 discloses a composition for treating liver spots which comprises tranexamic acid, vitamin C, vitamin B2 and/or vitamin B6. EP 1 002 515 A1 discloses a skin composition for external use for whitening and moisturizing skin which contains a first component selected from the group consisting of L-ascorbic acid and its derivatives, placenta extract, kojic acid and its derivatives, azelaic acid and its derivatives, glucosamine and its derivatives, hydroquinone glycosides and derivatives thereof, tranexamic acid and its derivatives and salicylic acid and its derivatives, and a second component selected from Lempuyang and solvent extracts thereof.

However, these agents were not always advantageous in terms of their effect and the like.

### DISCLOSURE OF THE INVENTION

The present invention provides a composition for whitening (whitening agent) having more superior effect.

As a result of intensive studies, the present inventors found that superior whitening effect can be obtained when tranexamic acid or a salt thereof is jointly used with L-cysteine, a derivative thereof or a salt thereof, and thus the present invention has been completed.

That is, the present invention relates to the followings.
(1) A composition which comprises (i) tranexamic acid or a salt thereof and (ii) L-cysteine, a derivative thereof or a salt thereof.
(2) A composition which comprises (i) 50 to 2,500 mg of tranexamic acid or a salt thereof and (ii) 30 to 750 mg of L-cysteine, a derivative thereof or a salt thereof, in terms of administrating amounts (doses) per day.
(3) A composition which comprises tranexamic acid and L-cysteine.
(4) A composition which comprises 50 to 2,500 mg of tranexamic acid and 30 to 750 mg of L-cysteine, in terms of administrating amounts (doses) per day.
(5) A composition which comprises (i) tranexamic acid or a salt thereof, (ii) L-cysteine, a derivative thereof or a salt thereof and (iii) L-ascorbic acid, a derivative thereof or a salt thereof
(6) A composition which comprises (i) 50 to 2,500 mg of tranexamic acid or a salt thereof, (ii) 30 to 750 mg of L-cysteine, a derivative thereof or a salt thereof and (iii) 50 to 3,000 mg of L-ascorbic acid, a derivative thereof or a salt thereof, in terms of administrating amounts (doses) per day.
(7) A composition which comprises tranexamic acid, L-cysteine and L-ascorbic acid.
(8) A composition which comprises 50 to 2,500 mg of tranexamic acid, 30 to 750 mg of L-cysteine and 50 to 3,000 mg of L-ascorbic acid, in terms of administrating amounts (doses) per day.
(9) The composition according to any one of (1) to (8), which is used for whitening.
(10) The composition according to any one of (1) to (8), which is used for preventing and/or treating pigmentations.
(11) The composition according to any one of (1) to (10), wherein its dosage form is an oral administration preparation.
(12) A method of whitening, which comprises administering (i) tranexamic acid or a salt thereof and (ii) L-cysteine, a derivative thereof or a salt thereof.
(13) A method for treating pigmentations, which comprises administering (i) tranexamic acid or a salt thereof and (ii) L-cysteine, a derivative thereof or a salt thereof.
(14) A method of whitening, which comprises administering (i) tranexamic acid or a salt thereof, (ii) L-cysteine, a derivative thereof or a salt thereof and (iii) L-ascorbic acid, a derivative thereof or a salt thereof.
(15) A method for treating pigmentations, which comprises administering (i) tranexamic acid or a salt thereof, (ii) L-cysteine, a derivative thereof or a salt thereof and (iii) L-ascorbic acid, a derivative thereof or a salt thereof.

### BEST MODE FOR CARRYING OUT THE INVENTION

Tranexamic acid (trans-4-aminomethylcyclohexanecarboxylic acid) or a salt thereof according to the present invention is a known compound, and regarding its obtaining method, a commercially available product may be used or it may be produced based on a known method. Examples of the salt of tranexamic acid include mineral acid salts such as hydrochloride, nitrate, and sulfate; organic acid salts such as methanesulfonate; alkali metal salts or alkaline earth metal salts such as sodium salt, potassium salt, calcium salt, and magnesium salt, and the like. According to the present invention, tranexamic acid is preferred as the tranexamic acid or a salt thereof.

The L-cysteine, a derivative thereof or a salt thereof according to the present invention is also a known compound, and as its obtaining method, a commercially available product may be used or it may be produced based on a known method. Examples of the derivatives of L-cysteine include N-acetyl-L-cysteine, L-homocysteine, L-cysteic acid, L-homocysteic acid, L-cysteine sulfinic acid, S-sulfino-L-cysteine, S-sulfo-L-cysteine, cystine (dimer of cysteine) and the like. In addition, examples of the salt of L-cysteine or a derivative thereof include mineral acid salts such as hydrochloride, nitrate, and sulfate; alkali metal salts or alkaline earth metal salts such as sodium salt, potassium salt, calcium salt, and magnesium salt, and the like. According to the present invention, L-cysteine is preferred as the L-cysteine, a derivative thereof or a salt thereof.

The L-ascorbic acid, a derivative thereof or a salt thereof according to the present invention is also a known compound, and regarding its obtaining method, a commercially available product may be used or it may be produced based on a known method. Examples of the salt of L-ascorbic acid or a derivative thereof include mineral acid salts such as hydrochloride, nitrate, and sulfate; alkali metal salts or alkaline earth metal salts such as sodium salt, potassium salt, calcium salt, and magnesium salt, and the like. Examples of the L-ascorbic acid, a derivative thereof or a salt thereof include L-ascorbic acid; L-ascorbic acid salts such as sodium L-ascorbate, magnesium L-ascorbate, potassium L-ascorbate, and calcium L-ascorbate; ascorbic acid monoalkyl or monoalkenyl esters such as L-ascorbic acid monostearate, L-ascorbic acid monopalmitate, and L-ascorbic acid monooleate; ascorbic acid dialkyl or dialkenyl esters such as L-ascorbic acid distearate, L-ascorbic acid dipalmitate, and L-ascorbic acid dioleate; ascorbic acid trialkyl or trialkenyl esters such as L-ascorbic acid tristearate, L-ascorbic acid tripalmitate, and L-ascorbic acid trioleate; L-ascorbyl sulfates such as L-ascorbyl sulfuric acid, sodium L-ascorbyl sulfate, potassium L-ascorbyl sulfate, magnesium L-ascorbyl sulfate, and calcium L-ascorbyl sulfate; L-ascorbyl phosphates such as L-ascorbyl phosphoric acid, sodium L-ascorbyl phosphate, potassium L-ascorbyl phosphate, magnesium L-ascorbyl phosphate, and calcium L-ascorbyl phosphate; and ascorbic acid glycosides such as L-ascorbic acid glucoside. According to the present invention, L-ascorbic acid is preferred as the L-ascorbic acid, a derivative thereof or a salt thereof.

The composition for whitening (whitening agent) of the present invention is administered to a person aiming at preventing and/or treating pigmentations such as chloasmas, freckles, sunburn, dark skin and melanoderma caused by a drug such as steroid.

The composition of the present invention may be further blended with known components which show whitening effect and components that reinforce whitening effect. Examples of these components include pantothenic acid, derivatives thereof or salts thereof (pantothenic acid; pantothenate, such as sodium pantothenate and calcium pantothenate; pantetheine; pantethine; phosphopantetheine; *etc*.), hydroquinone or derivatives thereof (hydroquinone; hydroquinone glucoside such as hydroquinone-β-D-glucose (arbutin); *etc*.), glucosamine or derivatives thereof (glucosamine; glucosamine esters such as acetylglucosamine; glucosamine ethers such as glucosamine methyl ether; *etc*.), hinokitiol or derivatives thereof (hinokitiol; hinokitiol glucoside such as hinokitiol glucoside; *etc*.), azelaic acid, derivatives thereof or salts thereof (azelaic acid; azelaic acid monoesters such as azelaic acid monoalkyl ester; azelaic acid diester such as azelaic acid dialkyl ester; *etc.*)*,* tocopherols (α-tocopherol, β-tocopherol, γ-tocopherol, δ-tocopherol, *etc.*), ubiquinones (coenzyme Q₆ (CoQ₆), coenzyme Q₇ (CoQ₇), coenzyme Q₈ (CoQ₈), coenzyme Q₉ (CoQ₉), coenzyme Q₁₀ (CoQ₁₀), *etc*.), carotenes (carotene, lutein, violaxanthin, spirilloxanthin, spheroidene, *etc*.), flavones (flavone, apigenin, luteolin, and glucosides thereof, *etc.*), isoflavone or derivatives thereof (isoflavone, isoflavone glucosides, *etc.*), flavanone or derivatives thereof (naringenin, eriodictyol, naringin, *etc*.), catechins (catechin, catechin gallate, gallocatechin, *etc.*), flavonols (kaempferol, quercetin, myricetin, and glucosides thereof, *etc*.), glycyrrhizic acid, derivatives thereof or salts thereof (glycyrrhizic acid; glycyrrhizinate such as dipotassium glycyrrhizinate and monoammonium glycyrrhizinate; *etc*.), glycyrrhetic acid, derivatives thereof or salts thereof (glycyrrhetic acid; glycyrrhetic acid alklyl ester such as stearyl glycyrrhetinate; *etc*.), kojic acid, derivatives thereof or salts thereof (kojic acid; kojic acid monoalkyl esters such as kojic acid monobutylate, kojic acid monocaprate, kojic acid monopalmitate and kojic acid monostearate; kojic acid dialkyl esters such as kojic acid dibutylate, kojic acid dipalimitate, kojic acid distearate and kojic acid dioleate; *etc*.), ellagic acid, derivatives thereof or salts thereof (ellagic acid; ellagic acid ethers such as ellagic acid tetramethyl ether; acyl derivatives of ellagic acid such as ellagic acid tetraacetate and ellagic acid tetrabenzoate; *etc*.), glutathione, derivatives thereof or salts thereof (glutathione; S-acylglutathiones such as S-lactoylglutathione; N,S-diacylglutathione diesters such as N,S-dioctanoylglutathione distearyl and N,S-dipalmitoylglutathione dicetyl; *etc*.), resorcinol or derivatives thereof (resorcinol; alkylated resorcinol such as 4-n-butylresorcinol, 4-isoamylresorcinol, 4-cyclohexylresorcinol and 5-methylresorcinol; halogenated resorcinol such as 4-chlororesorcinol and 4-bromoresorcinol; *etc*.), glycogen; coix seed, hamamelis (*Hamamelis mamelodis*), saxifrage (*Saxifraga stolonifera*), *Aquilaria agallocha,* char (*Thea sinensis*), Japanese knotweed (*Polygonum cuspidatum*), Melissa (*Melisa officinalis*), thyme (*Thymus vulgaris*), *Artemisia capillaris*, yarrow (*Achillea milefolium*), hypericum (*Hypericum erectum*), St. John's wort (*Hypericum perforatum*), peony (*Paeonia lactiflora*), peony (*Paeonia suffruticosa*), liquorice (*Glycyrrhiza glabra*), Glycyrrhiza (*Glycyrrhiza uralensis*), mulberry (*Morus bombycis*)*,* mulberry (*Morus alba*), *Morus australis, Sophora flavescens, Artetostaphylos uva-ursi, Dictyopteris prolifera, Dictyota linearis, Sargassum fusiforme, Lomentaria catenata,* white mountain heather (*Cassiope lycopodioides*), *Rhodymenia palmata, Sargassum ringgoldianum coreanum* (*Sargassum ringgoldianum*), *Sargassum yezoense, Sargassum confusum, Sphaerotrichia divaricata*, sundew (*Drosera rotundifolia*), *Drosera spatulata,* lavender (*Lavandula*), coptis rhizome (*Coptis japonicus*), Job's tears (*Coix lacryma-joli*), *Epigaea asiatica*, trailing arbutus (*Epigaea repens*), passion flower (*Passiflora incarnata*), passion fruit (*Passiflora edulis*), water lemon, passion flower (*Passiflora caerulea*), *Passiflora altebilobata, Passiflora moluccana* or *Passiflora cochinchinensis, Passiflora cupiformis*, wild pansy (*Viola tricolor*), sweet violet (*Viola odorata*), violet (*Violaceae*), *Viola japonica, Viola yedoensis, Viola verecunda, Viola diffusa, Viola patrinii, Viola acuminata*, Nepalese violet, *Viola patrini, Viola collina, Viola sororia, Viola grypoceras, Viola philippica, Viola vaginata, Viola verecunda, Clematis chinensis* Osbeck, clematis (*Clematis florida*), clematis (*Clematis patens*), virgin's bower (*Clematis terniflora*), hardy rubber tree (*Eucommia ulmoides*), *Euonymus trichocarpus* Hayata, *Euonymus oxyphyllus*, asparagus (*Asparagus officinalis*), *Polygonum bistorta* or *Bistorta major*, green pea (*Pisum sativum*), *Rosa multiflora, Scutellaria baicalensis, Ononis,* blackberry or raspberry (*Rubus*), *Sophora flavescens, Millettia reticulata, Acanthopanax gracilistylus, Asiasarum sieboldii,* hawthorn (*Crataegus cuneata*), *Cassia nomame,* white lily, *Inula japonica, Morus alba*, soybean, tea, extracts of Japanese Angelica (*Angelica acutiloba*), molasses, *Ampelopsis japonica Makino*, beech, grape seeds, Frode manita, hops, *Rosa rugosa, Chaenomeles sinensis* or *Pseudocydonia sinensis, Momordica grosvenori*, Aloe (*Aloe ferox*), *althea* (*Althaea*), arnica (*Arnica*), Umbelliferae (*Angelica keiskei*), *Artemisia capillaris,* nettle (*Urtica*), turmeric (*Curcuma*), *Phellodendron amurense*, German chamomile (*Matricaria chamomilla*), Japanese honeysuckle (*Lonicera japonica*), watercress (*Nasturtium officinale*), confrey (*Symphytum officinale*), sage (*Salvia*), *Lithospermum erythrorhizon*, beefsteak plant (*Perilla*), white birch (*Betula platyphylla*), pot marigold (*Calendula officinalis*), *Sambucus sieboldiana, Typha latifolia, Sapindus mukurossi*, milk-vetch (*Astragalus sinicus*), mugwort (*Artemisia*), eucalyptus (*Eucalyptus*), multiflora rose (*Rosa multiflora*), ginkgo (*Ginkgo biloba*), *Alnus,* wolfberry (*Lycium chinense*), *Micromelum minutum, Micromelum pubescens, Melothria indica, Mangifera indica, Melothria japonica,* raspberry (*Rubus idaeus*), firethorn (*Pyracantha fortuneana*) or extracts thereof; placenta extracts; and the like, although they are not limited to the above components. These components may be blended alone or as a combination of two or more thereof. Components other than the known components which show whitening effect and components that reinforce whitening effect may also be added to the composition for whitening (whitening agent) of the present invention. The blending amounts of these components are not particularly limited, so long as they do not spoil the whitening effect of the composition of the present invention.

The composition of the present invention may be orally or parenterally administered (dosed). Examples of the preparations which are orally administered (oral administration preparations) include dosage forms such as tablets, capsules, powders, fine granules, solutions, troches, and jellies. Examples of the preparations which are parenterally administered (parenteral administration preparations) include dosage forms such as extracts, hard cream preparations, spirits, suppositories, suspensions, tinctures, ointments, cataplasmas, liniments, lotions, aerosols, ophthalmic solutions, and injections, and dosage forms such as extracts, hard cream preparations, spirits, suspensions, tinctures, ointments, cataplasmas, liniments, lotions, and aerosols are preferred as the parenteral administration preparations. In addition, it is possible to make the composition for whitening of the present invention into an embodiment of cosmetic compositions such as lotion, cream, face lotion, milky lotion, foam preparations, foundation, pack preparations, skin lotion, shampoo, rinse, and conditioner.

Pharmaceuticals can be prepared by conventionally known preparation techniques, and appropriate pharmaceutical additives can be added to the pharmaceuticals. Examples of the pharmaceutical additives include excipients, binders, disintegrants, lubricants, glidants, suspending agents, emulsifiers, stabilizers, moisture keeping (moistening) agents, preservatives, solvents, solubilizers, antiseptics, flavoring substances, sweeteners, dyes, flavors, propellants and the like, and these pharmaceutical additives may be selected and added in appropriate amounts within such a range that they do not spoil the effects of the present invention.

Regarding the blending ratio of (i) tranexamic acid or a salt thereof and (ii) L-cysteine, a derivative thereof or a salt thereof in the composition of the present invention, an appropriate blending ratio may be determined by optionally carrying out examinations, but as a weight ratio, (i) : (ii) = 1 : 0.01 to 15 is preferred, 1 : 0.1 to 1.5 is more preferred, and 1 : 0.32 is most preferred. Also, regarding the blending ratio of (i) tranexamic acid or a salt thereof, (ii) L-cysteine, a derivative thereof or a salt thereof and (iii) L-ascorbic acid, a derivative thereof or a salt thereof in the composition of the present invention, an appropriate blending ratio may be determined by optionally carrying out examinations, but as a weight ratio, (i) : (ii) : (iii) = 1 : 0.01 to 15 : 0.01 to 60 is preferred, 1 : 0.1 to 1.5 : 0.1 to 6 is more preferred, and 1 : 0.32 : 0.4 is most preferred.

An appropriate administrating amount (dose) of the composition for whitening of the present invention may be determined by carrying out optional examinations in terms of sex, age and symptoms of each user, administrating (dosing) method, administrating (dosing) frequency, administrating (dosing) time and the like. For example, in the case of an internal use, it is preferable to formulate in such a manner that 50 to 2,500 mg per day of tranexamic acid or a salt thereof is administered (dosed), and it is more preferable to formulate in such a manner that from 400 to 2,000 mg is administered (dosed). Also, it is preferable to formulate L-cysteine, a derivative thereof or a salt thereof in such a manner that 30 to 750 mg per day of the compound is administered (dosed), and it is more preferable to formulate it in such a manner that 150 to 480 mg of the compound is administered (dosed). In addition, it is preferable to formulate L-ascorbic acid, a derivative thereof or a salt thereof in such a manner that 50 to 3,000 mg per day of the compound is administered (dosed), and it is more preferable to formulate it in such a manner that 300 to 2,000 mg of the compound is administered (dosed).

The composition of the present invention may be administered (dosed) by making it into a single pharmaceutical preparation containing all of the components concerned in the present invention, or each of the components concerned in the present invention may be made into separate pharmaceutical preparations to obtain a kit preparation in which simultaneous or sequential administration (dose) of these preparations is possible.

Although the present invention is described below in detail based on examples, the present invention should not be limited thereto.

### 1. Pigmentation inhibitory effect

### 1.1 Test methods

### 1.1.1 1 Examination of ultraviolet ray (UVB) irradiation time

Ultraviolet ray (UVB) irradiation time was examined using two female 6-week-old Kwl:A-1 brown guinea pigs (SPF). That is, one of the guinea pigs was fixed on the abdominal position, and three 2 cm × 2 cm square ultraviolet ray irradiating sites were arranged in bilateral symmetry with the dorsal midline between, thus six sites in total. By shading the body except for the ultraviolet ray irradiating sites, an ultraviolet ray (UVB) was irradiated from a distance of 40 cm using five SE lamps (wavelength 250 to 350 nm, FL20S·E, manufactured by TOSHIBA). By setting the irradiation time to 4, 6, 8, 10, 12 or 14 minutes, skin reaction of each irradiating site (the presence and degree of erythema) was observed on the next day.

Next, by further setting the irradiation times at intervals of 15 seconds between the minimum time when the skin reaction was observed and the maximum time when the skin reaction was not observed, eight irradiating sites were arranged on the other guinea pig in the same manner as in the former case and then the skin reaction was observed, thus setting the ultraviolet ray (UVB) irradiation time in the test to 11 minutes and 30 seconds and 11 minutes and 45 seconds.

### 1.1.2 Test samples

Test samples were dissolved in distilled water for injection to the following concentrations.

| | |
|---|---|
| Sample (1): | control (no addition) |
| Sample (2): | tranexamic acid 37.5 mg/ml |
| Sample (3): | L-cysteine 12 mg/ml |
| Sample (4): | ascorbic acid 15 mg/ml |
| Sample (5): | tranexamic acid 37.5 mg/ml + ascorbic acid 15 mg/ml |
| Sample (6): | tranexamic acid 37.5 mg/ml + L-cysteine 12 mg/ml |
| Sample (7): | L-cysteine 12 mg/ml + ascorbic acid 15 mg/ml |
| Sample (8): | tranexamic acid 37.5 mg/ml + L-cysteine 12 mg/ml + ascorbic acid 15 mg/ml |

### 1.1.3 Ultraviolet ray irradiation

Examination was made by using five female 7-week-old Kw1:A-1 brown guinea pigs (SPF) in one group. That is, each of the guinea pigs was fixed on the abdominal position using a fixation plate on the respective ultraviolet ray irradiating days (on the day of the commencement of sample administration and on the 2nd and 4th days thereafter). One 2 cm × 2 cm square arranged on the left or right side with the guinea pig dorsal midline between was used as the ultraviolet ray irradiating site, and the body was shaded except for the ultraviolet ray irradiating site. Two guinea pigs in one group were irradiated with an ultraviolet ray (UVB) for 11 minutes and 30 seconds, and three guinea pigs in one group, from a distance of 40 cm using five SE lamps (wavelength 250 to 350 nm, FL20S·E, manufactured by TOSHIBA). During the testing period (14 days), each test sample was orally administered twice a day. Each sample solution was administered at a dose of 10 ml/kg. In this case, the administration was carried out after the ultraviolet ray irradiation on each ultraviolet ray irradiation day, or after the judgment on each pigmentation judging day.

### 1.1.4 Judgment of pigmentation

Before the irradiation on the day of the commencement of test sample administration and on the final day of the test, a ΔL value (L value on the observation day - L value before irradiation) was calculated by measuring L value (lightness) of the irradiated site using a color-difference meter (CR-300, manufactured by MINOLTA CAMERA). The results are shown in Table 1 (larger ΔL values show higher effect)

**Table 1**

| | Day of commencement | Final day of the test | |
|---|---|---|---|
| | L value | L value | ΔL value |
| Sample (1) | 61.79 ± 1.33 | 54.68 ± 1.20 | -7.10 ± 1.45 |
| Sample (2) | 62.06 ± 0.83 | 58.30 ± 1.20 | -3.76 ± 1.78* |
| Sample (3) | 62.83 ± 1.55 | 59.52 ± 3.10 | -3.32 ± 1.70** |
| Sample (4) | 62.11 ± 0.81 | 57.35 ± 1.56 | -4.76 ± 1.62* |
| Sample (5) | 62.07 ± 0.96 | 58.62 ± 2.93 | -3.45 ± 2.28* |
| Sample (6) | 62.24 ± 0.62 | 59.54 ± 1.26 | -2.70 ± 1.56** |
| Sample (7) | 62.68 ± 1.03 | 56.18 ± 2.06 | -6.50 ± 1.65 |
| Sample (8) | 62.98 ± 1.75 | 60.79 ± 2.45 | -2.20 ± 1.00** |

| | | | |
|---|---|---|---|
| *: p<0.05, **: p<0.0 vs sample (1) | | | |

### 1.2 Results

As is evident from Table 1, the samples (6) and (8) concerned in the present invention showed excellent pigmentation inhibitory effect. That is, in comparison with the tranexamic acid-single administered group (sample (2) in the table) and the L-cysteine-single administered group (sample (3) in the table), the tranexamic acid- and L-cysteine-administered group (sample (6) in the table) showed excellent pigmentation inhibitory effect.

In addition, the L-cysteine- and ascorbic acid-administered group (sample (7) in the table) from which a certain degree of pigmentation inhibitory effect was expected showed no effect contrary to the expectation but rather showed an effect to accelerate pigmentation. This is evident when compared with the results of the L-cysteine-single administered group (sample (3) in the table) and ascorbic acid-single administered group (sample (4) in the table).

On the other hand, the group in which L-cysteine, ascorbic acid and tranexamic acid were administered (sample (8) in the table) not only turned the pigmentation acceleration of the sample (7)-administered group to pigmentation inhibition but also showed markedly excellent pigmentation inhibitory effect.

### 2. Formulation examples

### 2.1 Tablets

Tablets were produced in the usual way based on the following composition (6 tablets as daily dose).

| | |
|---|---|
| Tranexamic acid | 1,500 mg |
| L-Cysteine | 240 mg |
| Microcrystalline cellulose | 100 mg |
| Corn starch | 40 mg |
| Low substituted hydroxypropylcellulose | 50 mg |
| Hydroxypropylcellulose | 30 mg |
| Magnesium stearate | 20 mg |
| Hydroxypropylmethylcellulose 2910 | 60 mg |
| Macrogol 6000 | 12 mg |
| Talc | 10 mg |
| Titanium oxide | 18 mg |

### 2.2 Tablets

Tablets were produced in the usual way based on the following composition (6 tablets as daily dose).

| | |
|---|---|
| Tranexamic acid | 750 mg |
| L-Cysteine | 240 mg |
| L-Ascorbic acid | 300 mg |
| Microcrystalline cellulose | 200 mg |
| Corn starch | 100 mg |
| Low substituted hydroxypropylcellulose | 90 mg |
| Hydroxypropylcellulose | 30 mg |
| Magnesium stearate | 25 mg |
| Hydroxypropylmethylcellulose 2910 | 80 mg |
| Macrogol 6000 | 16 mg |
| Talc | 14 mg |
| Titanium oxide | 24 mg |

### 2.3 Tablets

Tablets were produced in the usual way based on the following composition (6 tablets as daily dose).

| | |
|---|---|
| Tranexamic acid | 750 mg |
| L-Cysteine | 160 mg |
| L-Ascorbic acid | 300 mg |
| α-Tocopherol | 300 mg |
| Microcrystalline cellulose | 170 mg |
| Corn starch | 200 mg |
| Low substituted hydroxypropylcellulose | 70 mg |
| Hydroxypropylcellulose | 30 mg |
| Magnesium stearate | 20 mg |
| Hydroxypropylmethylcellulose 2910 | 60 mg |
| Macrogol 6000 | 12 mg |
| Talc | 10 mg |
| Titanium oxide | 18 mg |

### 2.4 Tablets

Tablets were produced in the usual way based on the following composition (6 tablets as daily dose).

| | |
|---|---|
| Tranexamic acid | 1,000 mg |
| L-Cysteine | 480 mg |
| L-Ascorbic acid | 600 mg |
| Pyridoxine hydrochloride | 100 mg |
| Riboflavin | 30 mg |
| Microcrystalline cellulose | 200 mg |
| Corn starch | 950 mg |
| Hydroxypropylcellulose | 50 mg |
| Magnesium stearate | 25 mg |
| Hydroxypropylmethylcellulose 2910 | 75 mg |
| Macrogol 6000 | 15 mg |
| Talc | 11.5 mg |
| Titanium oxide | 22.5 mg |

### 2.5 Solutions

Solutions were produced in the usual way based on the following composition (100 ml as daily dose).

| | |
|---|---|
| Tranexamic acid | 1,000 mg |
| L-Cysteine | 240 mg |
| *Keishi-bukuryo-gan* extract | 3,750 mg |
| Sorbitol | 3,000 mg |
| Citric acid | 100 mg |
| Sodium citrate | 30 mg |
| Perfume | proper amount |
| Purified water | 100 ml |

### 2.6 Tablets

Tablets were produced in the usual way based on the following composition (6 tablets as daily dose).

| | |
|---|---|
| Tranexamic acid | 1,000 mg |
| L-Cysteine | 240 mg |
| Chamomile (*Matricaria chamomilla*) | 600 mg |
| Bearberry (*Arctostaphylos uva-ursi*) | 300 mg |
| Microcrystalline cellulose | 200 mg |
| Corn starch | 165 mg |
| Hydroxypropylcellulose | 50 mg |
| Magnesium stearate | 25 mg |
| Hydroxypropylmethylcellulose 2910 | 75 mg |
| Macrogol 6000 | 15 mg |
| Talc | 11.5 mg |
| Titanium oxide | 22.5 mg |

### 2.7 Tablets

Tablets were produced in the usual way based on the following composition (6 tablets as daily dose).

| | |
|---|---|
| Tranexamic acid | 1,000 mg |
| L-Cysteine | 240 mg |
| Ceramide | 20 mg |
| Microcrystalline cellulose | 150 mg |
| Corn starch | 145 mg |
| Hydroxypropylcellulose | 25 mg |
| Magnesium stearate | 10 mg |
| Hydroxypropylmethylcellulose 2910 | 42 mg |
| Macrogol 6000 | 7 mg |
| Talc | 168 mg |
| Titanium oxide | 7 mg |
| Sucrose | 660 mg |
| Acacia | 17 mg |
| Precipitated calcium carbonate | 150 mg |

### 2.8 Tablets

Tablets were produced in the usual way based on the following composition (6 tablets as daily dose).

| | |
|---|---|
| Tranexamic acid | 1,000 mg |
| L-Cysteine | 480-mg |
| Flavangenol | 30 mg |
| Collagen | 1,000 mg |
| Microcrystalline cellulose | 203 mg |
| Corn starch | 40 mg |
| Low substitution degree hydroxypropylcellulose | 70 mg |
| Hydroxypropylcellulose | 50 mg |
| Magnesium stearate | 27 mg |
| Hydroxypropylmethylcellulose 2910 | 80 mg |
| Macrogol 6000 | 16 mg |
| Talc | 14 mg |
| Titanium oxide | 24 mg |

### 2.9 Vanishing cream

A vanishing cream (100 g) was produced in the usual way based on the following composition.

| | |
|---|---|
| (A) Polysorbate 60 | 1 g |
| Polyoxyethylene sorbitol tetraoleate (60E.O.) | 0.5 g |
| Glyceryl monostearate (oleophilic type) | 1.0 g |
| Cetyl palmitate | 4.0 g |
| Paraffin wax (135°F) | 3.0 g |
| Stearic acid | 8.0 g |
| Behenyl alcohol | 2.0 g |
| Cetyl isooctanoate | 6.0 g |
| Butylparaben | 0.1 g |
| (B) Methylparaben | 0.1 g |
| 2% Sodium hydroxide aqueous solution | 4.0 g |
| 1,3-Butylene glycol | 7.0 g |
| Tranexamic acid | 1.0 g |
| L-Cysteine | 1.0 g |
| Purified water | balance |

### 2.10 Milky lotion

A milky lotion (100 g) was produced in the usual way based on the following composition.

| | | |
|---|---|---|
| (A) | Polysorbate 60 | 1 g |
| | Polyoxyethylene sorbitol tetraoleate (60E.O.) | 0.5 g |
| | Glyceryl monostearate (oleophilic type) | 1.0 g |
| | Stearic acid | 0.5 g |
| | Behenyl alcohol | 0.5 g |
| | Liquid paraffin | 4.0 g |
| | Glyceryl tri-2-ethylhexanoate | 4.0 g |
| | Cetyl isooctanoate | 2.0 g |
| | Butylparaben | 0.1 g |
| (B) | Methylparaben | 0.1 g |
| | Carboxyvinyl polymer (1% aqueous solution) | 5.0 g |
| | 1,3-Butylene glycol | 5.0 g |
| | Tranexamic acid | 1.0 g |
| | L-Cysteine | 1.0 g |
| | Magnesium L-ascorbylphosphate | 1.0 g |
| | With purified water to | 90.0 g |
| (C) | 1% Sodium hydroxide aqueous solution | 2.5 g |
| | Purified water | 7.5 g |
| (D) | Perfume | proper amount |

### 2.11 Lotion

A lotion (100 g) was produced in the usual way based on the following composition.

| | | |
|---|---|---|
| (A) | POE hydrogenated castor oil 60 | 1.0 g |
| | Perfume | proper amount |
| | Ethanol | 15.0 g |
| | Methylparaben | 0.1 g |
| (B) | Citric acid | 0.1 g |
| | Sodium citrate | 0.3 g |
| | 1,3-Butylene glycol | 4.0 g |
| | Tranexamic acid | 1.0 g |
| | L-Cysteine | 1.0 g |
| | Magnesium L-ascorbylphosphate | 1.0 g |
| | Purified water | balance |

### Industrial Applicability

The composition of the present invention showed excellent melanin pigment deposition inhibitory effect. Accordingly, the composition of the present invention is useful as a composition used for whitening (whitening agent) and also as a composition for the prevention and/or treatment of pigmentations such as chloasmas, freckles, sunburn, dark skin and melanoderma caused by a drug such as steroid.

## Claims

1. A composition which comprises (i) tranexamic acid or a salt thereof and (ii) L-cysteine, a derivative thereof or a salt thereof.

2. A composition according to claim 1 which comprises tranexamic acid and L-cysteine.

3. A composition according to claim 1 or 2 which comprises (i) 50 to 2,500 mg of tranexamic acid or a salt thereof and (ii) 30 to 750 mg of L-cysteine, a derivative thereof or a salt thereof, in terms of administrating amounts (doses) per day.

4. A composition which comprises (i) tranexamic acid or a salt thereof, (ii) L-cysteine, a derivative thereof or a salt thereof and (iii) L-ascorbic acid, a derivative thereof or a salt thereof.

5. A composition according to claim 4 which comprises tranexamic acid, L-cysteine and L-ascorbic acid.

6. A composition according to claim 4 or 5 which comprises (i) 50 to 2,500 mg of tranexamic acid or a salt thereof, (ii) 30 to 750 mg of L-cysteine, a derivative thereof or a salt thereof and (iii) 50 to 3,000 mg of L-ascorbic acid, a derivative thereof or a salt thereof, in terms of administrating amounts (doses) per day.

7. The composition according to any one of claims 1 to 6 , which is used for whitening.

8. The composition according to any one of claims 1 to 6 , which is used for preventing and/or treating pigmentations.

9. The composition according to any one of claims 1 to 8 , wherein its dosage form is an oral administration preparation.

10. The use of (i) tranexamic acid or a salt thereof and (ii) L-cysteine, a derivative thereof or a salt thereof for preparing a pharmaceutical composition for whitening.

11. The use of (i) tranexamic acid or a salt thereof and (ii) L-cysteine, a derivative thereof or a salt thereof for preparing a pharmaceutical composition for treating pigmentations.

12. The use according to claim 10 or 11 , wherein the composition additionally comprises (iii) L-ascorbic acid, a derivative thereof or a salt thereof.

## Patentansprüche

1. Zusammensetzung, umfassend (i) Tranexamsäure oder ein Salz davon und (ii) L-Cystein, ein Derivat davon oder ein Salz davon .

2. Zusammensetzung nach Anspruch 1, umfassend Tranexamsäure und L-Cystein.

3. Zusammensetzung nach Anspruch 1 oder 2, umfassend (i) 50 bis 2.500 mg Tranexamsäure oder ein Salz davon und (ii) 30 bis 750 mg L-Cystein, ein Derivat davon oder ein Salz davon, in Form von Tagesgaben (Dosen).

4. Zusammensetzung, umfassend (i) Tranexamsäure oder ein Salz davon, (ii) L-Cystein, ein Derivat davon oder ein Salz davon und (iii) L-Ascorbinsäure, ein Derivat davon oder ein Salz davon .

5. Zusammensetzung nach Anspruch 4, umfassend Tranexamsäure, L-Cystein und L-Ascorbinsäure.

6. Zusammensetzung nach Anspruch 4 oder 5, umfassend (i) 50 bis 2.500 mg Tranexamsäure oder ein Salz davon, (ii) 30 bis 750 mg L-Cystein, ein Derivat davon oder ein Salz davon und (iii) 50 bis 3.000 mg L-Ascorbinsäure, ein Derivat davon oder ein Salz davon, in Form von Tagesgaben (Dosen).

7. Verbindung nach einem der Ansprüche1 bis 6 zur Verwendung zum Aufhellen.

8. Verbindung nach einem der Ansprüche1 bis 6 zur Verwendung bei der Prävention und/oder Behandlung von Pigmentierungen.

9. Verbindung nach einem der Ansprüche1 bis 8, wobei die Dosierungsform ein Präparat zur oralen Verabreichung ist.

10. Verwendung von (i) Tranexamsäure oder eines Salzes davon und (ii) L-Cystein, eines Derivates davon oder eines Salzes davon zur Herstellung einer pharmazeutischen Zusammensetzung zum Aufhellen.

11. Verwendung von (i) Tranexamsäure oder eines Salzes davon und (ii) L-Cystein, eines Derivates davon oder eines Salzes davon Herstellung einer pharmazeutischen Zusammensetzung zur Behandlung von Pigmentierungen.

12. Verwendung nach Anspruch 10 oder 11, wobei die Zusammensetzung zusätzlich (iii) L-Ascorbinsäure, ein Derivat davon oder ein Salz davon umfasst.

## Revendications

1. Composition qui comprend (i) de l'acide tranexamique ou un de ses sels et (ii) de la L-cystéine, un de ses dérivés ou un de ses sels.

2. Composition selon la revendication 1 qui comprend de l'acide tranexamique et de la L-cystéine.

3. Composition selon la revendication 1 ou 2 qui comprend (i) 50 à 2 500 mg d'acide tranexamique ou d'un de ses sels et (ii) 30 à 750 mg de L-cystéine, d'un de ses dérivés ou d'un de ses sels, en termes de quantités à administrer (doses) par jour.

4. Composition qui comprend (i) de l'acide tranexamique ou un de ses sels, (ii) de la L-cystéine, un de ses dérivés ou un de ses sels et (iii) de l'acide L-ascorbique, un de ses dérivés ou un de ses sels.

5. Composition selon la revendication 4 qui comprend de l'acide tranexamique, de la L-cystéine et de l'acide L-ascorbique.

6. Composition selon la revendication 4 ou 5 qui comprend (i) 50 à 2 500 mg d'acide tranexamique ou d'un de ses sels, (ii) 30 à 750 mg de L-cystéine, d'un de ses dérivés ou d'un de ses sels et (iii) 50 à 3 000 mg d'acide L-ascorbique, d'un de ses dérivés ou d'un de ses sels, en termes de quantités à administrer (doses) par jour.

7. Composition selon l'une quelconque des revendications 1 à 6, laquelle est utilisée pour l'éclaircissage.

8. Composition selon l'une quelconque des revendications 1 à 6, laquelle est utilisée pour prévenir et/ou traiter les pigmentations.

9. Composition selon l'une quelconque des revendications 1 à 8, où sa forme posologique est une préparation pour administration orale.

10. Utilisation (i) d'acide tranexamique ou d'un de ses sels et (ii) de L-cystéine, d'un de ses dérivés ou d'un de ses sels pour la préparation d'une composition pharmaceutique destinée à l'éclaircissage.

11. Utilisation (i) d'acide tranexamique ou d'un de ses sels et (ii) de L-cystéine, d'un de ses dérivés ou d'un de ses sels pour la préparation d'une composition pharmaceutique destinée à traiter les pigmentations.

12. Utilisation selon la revendication 10 ou 11, où la composition comprend additionnellement (iii) de l'acide L-ascorbique, un de ses dérivés ou un de ses sels.
